Europäisches Patentamt
European Patent Office
Office européen des brevets

(19)

(11) Veröffentlichungsnummer: **0 411 409 A1**

(12) # EUROPÄISCHE PATENTANMELDUNG


(21) Anmeldenummer: **90113915.4**

(51) Int. Cl.5: **C07C 69/708**, C07C 67/31

(22) Anmeldetag: **20.07.90**

(30) Priorität: **29.07.89 DE 3925256**

(43) Veröffentlichungstag der Anmeldung: **06.02.91 Patentblatt 91/06**

(84) Benannte Vertragsstaaten: **BE CH DE FR GB IT LI NL**



(71) Anmelder: **BASF Aktiengesellschaft**
**Carl-Bosch-Strasse 38**
**D-6700 Ludwigshafen(DE)**

(72) Erfinder: **Hickmann, Eckhard, Dr.**
**Kantstrasse 23**
**D-6701 Dannstadt-Schauernheim(DE)**


(54) **Substituierte 3-Oxipropionsäure-tert.-butylester.**


(57) Substituierte 3-Oxipropionsäure-tert.-butylester der allgemeinen Formel I
$R[-O-(CH_2)_2-COOC(CH_3)_3]_n$ (I)
in der
R Alkyl mit 2, 3 oder 5 bis 20 C-Atomen, Alkandiyl mit 2 bis 20 C-Atomen, Alkantriyl mit 3 bis 20 C-Atomen, Alkantetryl mit 4 bis 20 C-Atomen, Alkenyl mit 3 bis 20 C-Atomen, Alkendiyl mit 4 bis 20 C-Atomen, Alkinyl mit 3 bis 20 C-Atomen, Alkindiyl mit 4 bis 20 C-Atomen, Polyether-α,ω-diyl der allgemeinen Formel

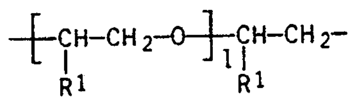

(II)

in der $R^1$ für Wasserstoff und/oder Methyl und l für eine ganze Zahl von 4 bis 400 steht, bedeutet, wobei die Reste R durch ein bis fünf Substituenten aus der Gruppe Halogen, Hydroxi, Alkoxi, Alkoxialkyl, Acyloxi, Trialkylsilyl, Thiol, Thio, sec.-Amino, tert.-Amino, Oximether, Nitro, Halogenalkyl, Aldehyd, Acyl, Ketal, Ester, Nitril, gegebenenfalls substituiertes Aryl und aromatische oder nichtaromatische Heterocyclen substituiert sein können, und
n 1 bis 4 bedeutet.


EP 0 411 409 A1

## SUBSTITUIERTE 3-OXIPROPIONSÄURE-TERT.-BUTYLESTER

Die vorliegende Erfindung betrifft neue substituierte 3-Oxipropionsäuretert.-butylester und Verfahren zu dern Herstellung.

Die Addition von Alkoholen an Acrylester zu substituierten 3-Oxipropionsäureestern (oxicarbonylethylethern) unter Katalyse von Alkoholaten ist z.B. aus J. Chem. Soc. 1891, 59, 474-476 bekannt. Dort wird die Reaktion von Alkoholen an Acrylester mit identischer Alkoholkomponente, z.B. zur Synthese von 3-Methoxipropionsäuremethylester aus Methanol und Acrylsäuremethylester oder zur Herstellung von 3-Ethoxipropionsäureethylester aus Ethanol und Acrylsäureethylester durchgeführt.

Falls jedoch der zu addierende Alkohol nicht identisch ist mit dem Alkoholteil im eingesetzten Acrylester, so erhält man bei ebenfalls alkoholatkatalysierter Reaktion durch Umesterungen stets Gemische unterschiedlicher Ester (Kirk-Othmer, "Encyclopedia of Chemical Technology", 3. Aufl. 1978, Bd. 1, S. 336). Noch komplexere Reaktionsgemische erhält man beim Einsatz von mehrwertigen Alkoholen. Andere Basen als Alkalialkoholate, z.B. Aluminiumalkoholate, konz. wäßrige Natronlauge oder Benzyl-trimethylammoniumhydroxid eignen sich nach dem Stand der Technik für die basenkatalysierte Addition von Alkoholen an Acrylester nicht (C.E. Rehberg et al., J. Amer. Chem. Soc. 1946, 68, 544; 1947, 69, 2966; 72, 2205; Kirk-Othmer, l.c.).

Aus J. Amer. Chem. Soc. 1947, 69, 2328-2335 ist zusätzlich bekannt, daß Benzyl-trimethylammoniumhydroxid sich zur Addition von Hydroxiethylthio-Verbindungen an Acrylnitril eignet, bei der Addition dieser Alkohole an Acrylester jedoch ebenfalls versagt.

Ferner ist aus Journal f. prakt. Chemie 1986, 328, 643-647 bekannt, daß die mangelhafte Selektivität bei der Addition von Alkoholen an Acrylester auch durch die Verwendung eines Katalysators aus Nickel(II)-acetylacetonat und n-Butyllithium nicht behoben wird.

Es bestand daher die Aufgabe, substituierte 3-Oxipropionsäureester zu finden, die ein selektives Verfahren für die genannte Alkylierungsreaktion ermöglichen.

Demgemäß wurden die neuen substituierten 3-Oxipropionsäure-tert.-butylester der allgemeinen Formel I

$R[-O-(CH_2)_2-COOC(CH_3)_3]_n$ (I)

in der

R Alkyl mit 2, 3 oder 5 bis 20 C-Atomen, Alkandiyl mit 2 bis 20 C-Atomen, Alkantriyl mit 3 bis 20 C-Atomen, Alkantetryl mit 4 bis 20 C-Atomen, Alkenyl mit 3 bis 20 C-Atomen, Alkendiyl mit 4 bis 20 C-Atomen, Alkinyl mit 3 bis 20 C-Atomen, Alkindiyl mit 4 bis 20 C-Atomen, Polyether-$\alpha,\omega$-diyl der allgemeinen Formel

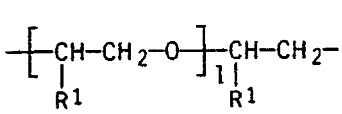

(II)

in der $R^1$ für Wasserstoff und/oder Methyl und l für eine ganze Zahl von 4 bis 400 steht, bedeutet, wobei die Reste R durch ein bis fünf Substituenten aus der Gruppe Halogen, Hydroxi, Alkoxi, Alkoxialkyl, Acyloxi, Trialkylsilyl, Thiol, Thio, sec.-Amino, tert.-Amino, Oximether, Nitro, Halogenalkyl, Aldehyd, Acyl, Ester, Nitril, gegebenenfalls substituiertes Aryl und aromatische oder nichtaromatische Heterocyclen substituiert sein können, und

n 1 bis 4 bedeutet,

gefunden, sowie ein Verfahren zu deren Herstellung.

Der Rest R in Formel I hat im einzelnen folgende Bedeutungen:

- $C_2$-, $C_3$-, $C_5$- bis $C_{20}$-Alkyl wie Ethyl, n-Propyl, iso-Propyl, n-Pentyl, bevorzugt $C_6$- bis $C_{20}$-Alkyl, besonders bevorzugt $C_6$- bis $C_{12}$-Alkyl wie n-Hexyl, n-Heptyl, n-Octyl, 2-Ethylhexyl, n-Decyl und n-Dodecyl,
- $C_2$- bis $C_{20}$-Alkandiyl, bevorzugt $C_2$- bis $C_{12}$-Alkandiyl, besonders bevorzugt $C_2$- bis $C_8$-Alkandiyl wie Ethan-1,2-diyl, Propan-1,2-diyl, Propan-1,3-diyl, n-Butan-1,4-diyl, n-Butan-1,2-diyl, n-Pentan-1,5-diyl, 2,2-Dimethyl-propan-1,3-diyl, n-Hexan-1,6-diyl und n-Octan-1,8-diyl,
- $C_3$- bis $C_{20}$-Alkantriyl, bevorzugt $C_3$- bis $C_{12}$-Alkantriyl, besonders bevorzugt $C_3$- bis $C_8$-Alkantriyl, wie Propan-1,2,3-triyl, n-Butan-1,2,4-triyl und n-Pentan-1,2,5-triyl,
- $C_4$- bis $C_{20}$-Alkantetryl, bevorzugt $C_4$- bis $C_{12}$-Alkantetryl, besonders bevorzugt $C_4$- bis $C_8$-Alkantetryl wie n-Butan-1,2,3,4-tetryl und 2,2-Dimethylpropan-1,3,4,5-tetryl,
- $C_3$- bis $C_{20}$-Alkenyl, bevorzugt $C_3$- bis $C_{16}$-Alkenyl, besonders bevorzugt $C_3$- bis $C_{12}$-Alkenyl wie Allyl, But-2-en-1-yl, But-3-en-1-yl, 3-Methyl-but-2-en-1-yl, 3-Methyl-but-3-en-1-yl, Hex-5-en-1-yl, Oct-7-en-1-yl und

Dec-9-en-1-yl,

- $C_4$- bis $C_{20}$-Alkendiyl, bevorzugt $C_4$- bis $C_{12}$-Alkendiyl, besonders bevorzugt $C_4$- bis $C_8$-Alkendiyl wie But-2-en-1,4-diyl, sut-3-en-1,2diyl, 2-Methyl-prop-2-en-1,4-diyl und Hex-3-en-1,6-diyl,
- $C_3$- bis $C_{20}$-Alkinyl, bevorzugt $C_3$- bis $C_{12}$-Alkinyl, besonders bevorzugt $C_3$- bis $C_8$-Alkinyl wie Prop-2-in-1-yl und Hex-5-in-1-yl,
- $C_4$- bis $C_{20}$-Alkindiyl, bevorzugt $C_4$- bis $C_{12}$-Alkindiyl, besonders bevorzugt $C_4$- bis $C_8$-Alkindiyl wie But-2-in-1,4-diyl und Hex-3-in2,5-diyl,
- $C_8$- bis $C_{800}$-Poly-(oxi-1,2-ethandiyl), bevorzugt $C_8$- bis $C_{400}$-Poly-(oxi-1,2-ethandiyl),
- $C_{12}$- bis $C_{1200}$-Poly-(oxi-1-methyl-1,2-ethandiyl), bevorzugt $C_{12}$- bis $C_{600}$-Poly-(oxi-1-methyl-1,2-ethandiyl).

Die zuvor genannten Reste können gegebenenfalls durch einen bis fünf, bevorzugt einen bis drei, besonders bevorzugt einen Substituenten aus der Gruppe tragen:

- Halogen, wie Fluor, Chlor, Brom und Jod, bevorzugt Fluor, Chlor und Brom, besonders bevorzugt Fluor und Chlor,
- Hydroxi,
- Alkoxi, wie $C_1$- bis $C_{20}$-Alkoxi, bevorzugt $C_1$- bis $C_{10}$-Alkoxi, besonders bevorzugt $C_1$- bis $C_6$-Alkoxi, wie Methoxi, Ethoxi, n- und iso-Propoxi, n-, iso-, sec.- und tert.-Butoxi, Hexoxi und Cyclohexoxi,
- Alkoxialkyl, wie $C_2$- bis $C_{20}$-Alkoxialkyl, bevorzugt $C_2$- bis $C_{12}$-Alkoxialkyl, besonders bevorzugt $C_2$- bis $C_8$-Alkoxialkyl wie Methoximethyl, Ethoximethyl, n- und iso-Propoximethyl, n-, iso-, sec.- und tert.-Butoximethyl und, n-Hexyloximethyl,
- Aryloxi, wie Phenoxi,
- Trialkylsilyl, wie Trimethylsilyl
- Thiol wie 2-Mercaptoethyl,
- Thio, wie 3-Thiopentan-1,5-diyl,
- sec.-Amino mit 2 bis 20 C-Atomen, bevorzugt mit 2 bis 16 C-Atomen, besonders bevorzugt mit 2 bis 14 C-Atomen, wie N,N-Dimethylamino, N,N-Diethylamino, N,N-Dipropylamino, N-t-Butyl-N-methylamino, N,N-Diallylamino, N-Allyl-N-methylamino, N-Allyl-N-ethylamino, N-2-Hydroxiethyl-N-methylamino, N-Phenyl-N-2-Hydroxiethylamino, N-Phenyl-N-methylamino und N,N-Dibenzylamino,
- Oximether mit 1 bis 6 C-Atomen, wie Methoxiimino, Ethoxiimino, Allyloxiimino, Cyclohexoxiimino und Benzyloxiimino,
- Nitro,
- Halogenalkyl wie $C_1$- bis $C_{20}$-Halogenalkyl, bevorzugt $C_1$- bis $C_8$-Fluor-und/oder Chloralkyl, besonders bevorzugt $C_1$- bis $C_4$-Fluor- und/oder Chloralkyl, wie Fluormethyl, Difluormethyl, Trifluormethyl und Trichlormethyl,
- Aldehyd mit 1 bis 12 C-Atomen wie Formyl, Formylmethyl und 2-Ethylhexanal-6-yl,
- Acyl, mit 2 bis 12 C-Atomen wie Acetyl, Propionyl, Pivaloyl, Acetylmethyl und Propionylmethyl,
- Ester wie $C_2$- bis $C_{20}$-Alkoxicarbonyl, bevorzugt $C_2$- bis $C_{12}$-Alkoxicarbonyl, besonders bevorzugt $C_2$- bis $C_6$-Alkoxicarbonyl, wie Methoxicarbonyl, Ethoxicarbonyl, n- und iso-Propoxicarbonyl, N-, iso-, sec.- und tert.-Butoxicarbonyl und tert.-Butoxicarbonylethyl,
- Nitril mit 1 bis 10 C-Atomen, wie Cyano, Cyanomethyl, 2-Cyanoethyl, 4-Cyanobutyl, 6-Cyanohexyl und 8-Cyanooctyl,
- ggf. substituiertes Aryl wie Phenyl, 1-Naphthyl und 2-Naphthyl, bevorzugt Phenyl und substituiertes Phenyl, wie o-, m-, p-Fluorphenyl, o-, m-, p-Chlorphenyl, o-, m- und p-Nitrophenyl,

Der Rest $R^1$ in Formel II bedeutet Wasserstoff und Methyl, bevorzugt Wasserstoff und der Index l eine ganze Zahl von 4 bis 400, bevorzugt von 4 bis 200, besonders bevorzugt von 4 bis 50.

Die Herstellung der Verbindungen I und 11 kann wie folgt durchgeführt werden:

Die Vermischung der Reaktionskomponenten kann kontinuierlich, z.B. in einer Rührkesselkaskade oder in einem Schlaufenreaktor oder diskontinuierlich in einem Rührkessel erfolgen. Sei diskontinuierlicher Fahrweise legt man den ggf. gelösten Alkohol ganz oder teilweise vor und dosiert den Acrylester und ggf. den restlichen Alkohol getrennt oder als Lösung gemeinsam zu.

Die Reaktion wird üblicherweise im Temperaturbereich von ca. 0°C bis ca. 100 ° C, bevorzugt bei ca. 20°C bis ca. 40°C durchgeführt.

Das Mengenverhältnis von Acrylester zu Alkohol ist an sich beliebig. Um jedoch einen quantitativen Umsatz zu erzielen, setzt man vorzugsweise ca. 1,0 bis 1,5 Mol Acrylester pro Val Alkohol bzw. pro Val additionsfähiger Gruppe ein.

Man kann den Acrylester auch gezielt im Unterschuß einsetzen, falls partieller Umsatz gewünscht wird. Aus mehrwertigen Alkoholen erhält man insbesondere dann leicht Addukte mit definierter Zusammensetzung, wenn sterische Hinderung besteht, z.B. beim Neopentylglykol, oder wenn vicinale Alkoholgruppen vorhanden sind, z.B. beim Ethylenglykol oder beim Propylen1,2-glykol.

Die Additionsreaktion kann in Gegenwart von organischen Lösungsmitteln erfolgen, wie z.B. aliphatischen oder aromatischen Kohlenwasserstoffen, Ethern, tert.-Aminen, Ketonen, Estern, Nitrilen, Lactamen oder Harnstoffen; auch eignen sich andere Lösungsmittel, z.B. Alkohole, sofern ihr pKA-Wert ≧ ca. 18 ist. Am vorteilhaftesten arbeitet man jedoch lösungsmittelfrei.

Die Reaktion kann unter allen nichtsauren Schutzgasen durchgeführt werden; ein Schutzgas ist jedoch nicht erforderlich.

Die nachfolgend aufgeführten Katalysatoren werden gewöhnlich in Anteilen von 0,01 bis 10 Mol%, vorzugsweise in 0,5 - 5 Mol% bezogen auf den Alkohol, eingesetzt. Man kann sie wahlweise zu Beginn der Reaktion ganz oder teilweise mit vorlegen und/oder während der Reaktion kontinuierlich oder diskontinuierlich zudosieren. Manchmal ist es vorteilhaft, zum Erzielen eines quantitativen Umsatzes Katalysator nachzudosieren.

Geeignete quartär-Ammonium oder quartär-Phosphonium-hydroxide sind insbesondere solche, die sich in organischen Lösungsmitteln wie z.B. aliphatischen und aromatischen Kohlenwasserstoffen, Ethern, Alkoholen, tert. Aminen, Ketonen, Estern, Nitrilen, Lactamen oder Harnstoffen gut lösen. Beispielsweise sind Tetrapropyl-, Tetra-n-butyl-, Trioctylmethyl-, Dodecyltrimethyl-, Didodedecyldimethyl-, Hexadecyltrimethyl-, Octadecyltrimethyl-, Benzyltrimethyl-, Benzyltriethyl-, Dibenzyldimethylammoniumhydroxid, N-Dodecyl- und 4-Dimethylamino-N-hexadecyl-pyridiniumhydroxid, Trioctylmethyl-, Hexadecyltrimethyl- und Triphenylmethylphosphoniumhydroxid gut geeignet. Diese lipophilen Oniumhydroxide kann man als solche einsetzen oder man kann sie in situ in Gegenwart eines Metallhydroxides, vorzugsweise eines Alkalimetallhydroxides wie z.B. Natrium- oder Kaliumhydroxid, oder in Gegenwart eines stärker hydrophilen Oniumhydroxides wie z.B. Tetramethylammoniumhydroxid oder Cholin, aus mehr oder weniger lipophilen Oniumsalzen erzeugen. Insbesondere die mögliche in-situ-Herstellung von katalytisch ausgezeichnet wirksamen Ammonium- und Phosphonium-hydroxiden aus der Vielzahl technisch leicht zugänglicher quartär-Ammonium- und quartär-Phosphoniumhalogenide (-chloride, bromide) -methosulfate, -hydrogensulfate, -sulfate, -formiate und hydrogencarbonate macht das erfindungsgemäße Verfahren besonders breit anwendbar. Als Co-Katalysatoren kann man Alkoholate und/oder Komplexierungsmittel für Kationen, z.B. Kronenether, Polyethylenglykolether oder TDA-1® zusetzen.

Geeignete Acrylester sind vor allem die technisch zugänglichen Acrylsäure-und Methacrylsäureester, wie Acrylsäure-methylester, -ethylester, n- und iso-butyl-, 2-ethylhexylester, Methacrylsäuremethylester und insbesondere Acrylsäure-tert.-butylester. Alle diese Ester werden normalerweise in stabilisierter Form eingesetzt, können aber auch in Abwesenheit von Stabilisatoren verwendet werden. Eine zusätzliche Stabilisierung mit aromatischen Aminen (vgl. C.E. Rehberg, l.c.) ist nicht erforderlich.

Für die erfindungsgemäße, hochselektive Addition von Alkoholen an Acrylester eignet sich eine breite Palette von ein- und mehrwertigen Alkoholen. Geeignet sind z.B. aliphatische, gesättigte, einwertige Alkohole wie z.B. Methanol, Ethanol, n-Propanol, i-Propanol, n-Butanol, i-Butanol, sec-Butanol, n-Pentanol, i-Pentanol, neo-Pentanol, 2-Ethylhexanol-1 und höhere Homologe, cycloaliphatische, einwertige Alkohole wie z.B. Cyclopropanol, Cyclobutanol, Cyclopentanol, Cyclohexanol, Cyclooctanol, Cyclododecanol, aliphatische, ungesättigte, einwertige Alkohole wie z.B. Allylalkohol, Buten-2-ol-1, Buten-3-ol-1, 3-Methyl-buten-2-ol-1, 3-Methyl-buten-3-ol-1, Hexen-5-ol-1, Octen-7-ol-1, Decen-9-ol-1, Citronellol, Propargylalkohol, aromatisch substituierte Alkohole wie z.B. Benzylalkohol, 3-Methylbenzylalkohol, 4-Methoxibenzylalkohol, 4-tert.-Butoxibenzylalkohol, 3,4-Methylendioxibenzylalkohol, 4-Benzyloxibenzylalkohol, 2-Fluorbenzylalkohol, 4-Chlorbenzylalkohol, 2-Nitrobenzylalkohol, 4-Dimethylaminobenzylalkohol, 4-Methoxicarbonylbenzylalkohol, 2-Phenylethanol, 2-(4-Methoxiphenyl)-ethanol, Zimtalkohol, substituierte, aliphatische, einwertige Alkohole, z.B. Fluoralkanole wie 2,2,2-Trifluorethanol, Chloralkanole wie 6-Chlorhexanol, Bromalkanole wie 4-Brombutanol, Alkoxialkanole wie Methoxiethanol, 2-Methoxipropanol und höhere Homologe, ungesättigte Etheralkanole wie z.B. 4-Vinyloxibutanol, 6-Vinyloxihexanol, 8-Vinyloxioctanol, 2-Allyloxiethanol, 4-Allyloxibutanol, 6-Methallyloxihexanol, 2-Benzyloxiethanol, 2-Phenylethoxiethanol, Schwefel-substituierte Alkanole wie z.B. S-Ethylthioethanol, S-Phenylthioethanol, Stickstoff-substituierte Alkanole wie z.B. Dimethylaminoethanol, 3-Dimethylaminopropanol, 4-Dimethylaminobutanol, 3-Dimethylamino-2,2-dimethylpropanol, 6-Dimethylaminohexanol, Diethylaminoethanol, Di-n-butylaminoethanol, N-Phenyl-N-ethylaminoethanol, N-(3-Methylphenyl)-N-ethylaminoethanol, 4-Cyanobutanol, 6-Cyanohexanol, 8-Cyanooctanol, Phosphor-substituierte Alkanole, z.B. Hydroxiethylphosphonsäurediethylester, Silizium-substituierte Alkohole, z.B. 2-(Trimethylsilyl)-ethanol, heterocyclisch substituierte einwertige Alkohole, z.B 2-Hydroximethyl-tetrahydrofuran, 3-Hydroximethyltetrahydrofuran, 2-Hydroximethylfuran, 3-Hydroximethylfuran, 2,5-Dihydro-2,5-dimethoxi-2-hydroximethylfuran, 2,2-Dimethyl-4-hydroximethyldioxolan, 4-Hydroximethyl-tetrahydropyran, 2-Hydroximethylthiophen, N-Hydroxiethylethylenharnstoff, 5-Hydroximethyl-3-ethylisoxazol, N-Hydroxiethylmorpholin, 2-Hydroximethylpyridin, 2-Hydroximethylpyrazin, aliphatische, gesättigte, mehrwertige Alkohole, z.B. Ethylenglykol, 1,2-Propylenglykol, 1,3-Propylenglykol, Butan-1,4-diol, Butan-1,2-diol, Pentan-1,5-diol, 2,2-Dimethyl-propan-1,3-diol, Hexan-1,6-diol, Octan-1,8-diol, Glycerin, Pentan-1, 2, 5-triol,
cycloaliphatische, mehrwertige Alkohole, z.B. 1,4-Bis-hydroximethylcyclohexan, 1,3-Bis-hydroximethylcyclohexan, aliphatische, ungesättigte, mehrwertige Alkohole wie z.B. Buten-2-diol-1,4, Buten-3-diol-1,2, 2-Hydroximethyl-propen-2-ol, Hexen-3-diol-1,6, Butin-2-diol-1,4, Hexin-3-diol-2,5, aromatisch substituierte, mehrwertige Alkohole, z.B. 1,4-Bis-hydroximethylbenzol, heteroatomsubstituierte, mehrwertige Alkohole wie z.B. Diethylenglykol, Dibutylenglykol, Hydroxipivalinsäureneopentylglykolester, Thiodiethanol, N-Methyldiethanolamin, N-n-Butyl-diethanolamin, N-tert.-Butyldiethanolamin, N-Phenyl-diethanolamin, N-(3-Methylphenyl)-diethanolamin, N-(3-Chlorphenyl)-diethanolamin, 3-(N,N-Diethylamino)-propandiol-1,2, Triethanolamin, sowie heterocyclisch substituierte, mehrwertige Alkohole wie z.B. N,N′-Bis-hydroxiethylpiperazin oder 2-Isopropyl-3,4-bis-hydroximethylimidazol.

Geeignete Alkohole sind ferner solche, in denen zwei oder mehrere gleiche oder verschiedene funktionelle Gruppen zusätzlich zu den Alkoholfunktionen vorhanden sind, z.B. eignen sich Geraniol (zwei C-C-Doppelbindungen), ein-und mehrwertige Oligo- und Polyetheralkohole, z.B. Diethylenglykol-monomethylether (zwei Etherfunktionen), Triethylenglykol-monomethylether (drei Etherfunktionen), Triethylenglykol, Tripropylenglykol und Tributylenglykol (je zwei Etherfunktionen), Polyethylenglykole und ihre Monoether, Polypropylenglykole und ihre Monoether, sowie gemischte Polyethylen/propylenglykole und ihre Monoether (viele Etherfunktionen), Aminoalkoxialkohole, z.B. 2-(N,N-Dimethylaminoethoxi)-ethanol (Amino- und Etherfunktion), Esteraminoalkohole, z.B. 2-[N-(2′-tert.-Butoxicarbonylethyl)-N-methyl]-ethanol (Amino- und Esterfunktion). Diese Auflistung ist weit entfernt davon, vollständig zu sein; es soll nur das Prinzip veranschaulichen.

Weiterhin geeignet sind ein- und mehrwertige Alkohole, die zusätzlich solche funktionellen Gruppen tragen, die selber an Acrylester addieren, z.B. Aminoalkohole mit primären und/oder sekundären Aminogruppen, z.B. Ethanolamin, N-Methylethanolamin, 2-Aminopropanol-1, Neopentanolamin, 2-(2′-Aminoethyl)-aminoethanol, Diethanolamin, N-Phenylaminoethanol oder z.B. Alkohole mit Thiolgruppen, z.B. 2-Mercaptoethanol.

Bei Verwendung von Alkoholen mit zusätzlichen, stärker nucleophilen Gruppen wie z.B. primären und/oder sekundären Aminogruppen und/oder Thiolgruppen ist es oft vorteilhaft, die kontinuierliche oder diskontinuierliche Additionsreaktion zweistufig durchzuführen, wobei man zweckmäßigerweise in der ersten Stufe die stärker nucleophile Gruppe ohne Katalysator addiert und in der zweiten Stufe die Alkoholgruppe in Gegenwart einer der erfindungsgemäßen Katalysatoren addiert. Auf diese Weise kann man auch leicht N,O- oder S,O-Bisaddukte an unterschiedliche Acrylester herstellen, z.B. liefert die unkatalysierte Addition von N-Methylaminoethanol an Acrylsäuremethylester in der ersten Stufe 2-[N-(2′-Methoxicarbonylethyl)-N-methyl]-ethanol, das man in einer zweiten, katalysierten Stufe an z.B. Acrylsäure-tert.-butylester zum 3-[2′-N-(2″-Methoxicarbonylethyl)-N-methylamino-ethoxi]-propionsäure-tert.-butylester addieren kann. Auf diese Weise kann man leicht Addukte mit unterschiedlich reaktiven Estergruppen erzeugen.

Häufig können die Reaktionsprodukte ohne weitere Reinigung oder sonstige Aufarbeitung in eine evtl. Folgestufe eingesetzt werden. Als Reinigungsmethode genügt in der Regel das Auswaschen des Katalysators mit Wasser und das Abziehen der Niedersieder. Vor einer destillativen Reinigung wird der Katalysator zweckmäßigerweise vorher neutralisiert.

Die substituierten 3-Oxipropionsäure-tert.-butylester I eignen sich z.B. als Riechstoffe, Extraktionsmittel, Schmiermittel und Schmiermittel-Zusatzstoffe (vgl. Ullmann, Enzyklopädie der technischen Chemie, 4. Aufl., 1976, Bd. 11, S. 89), als Lösungsmittel (vgl. Ullmann, 4. Aufl., 1978, Bd. 16, S. 279f, insbes. S. 301), als Weichmacher (vgl. Ullmann, 4. Aufl, 1983, S. 349f, insbes. S. 367), als Vorprodukte für Polyester (vgl. Ullmann, 4. Aufl., 1980, Bd. 19, S. 61). Die Amin-substituierten 3-Oxipropionsäure-tert.-butylester I eignen sich auch als organische Basen und basische Katalysatoren.

Die folgenden Beispiele illustrieren die Erfindung:

Beispiel 1

Benzyl-trimethylammoniumhydroxid-katalysierte Addition von Ethanol an Acrylsäure-tert.-butylester

Identität und Reinheit aller Addukte wurden $^1H$-NMR-spektroskopisch sichergestellt (Lösungsmittel: $CDCl_3$, innerer Standard: TMS). Alle Addukte weisen neben zusätzlichen NMR-Banden einheitlich folgende charakteristische Banden im Intensitätsverhältnis 9:2:2 auf:

δ = 1,4 bis 1,45 ppm (s,tert.-Butoxicarbonylgruppe(n)), 2,45 bis 2,6 ppm (t, Methylengruppe(n) in α-Stellung zur (zu den) tert.-Butoxicarbonylgruppe(n)), 3,6 bis 3,9 ppm (t, Methylengruppe(n) in ß-Stellung zur (zu den) tert.-Butoxicarbonylgruppe(n)).

Zum vorgelegten Gemisch aus 46 g Ethanol und 8 g Benzyl-trimethylammoniumhydroxid tropft man unter Rühren innerhalb von ca. 3 h bei 20°C 154 g Acrylsäure-tert.-butylester und läßt noch 4 h bei ca. 20°C nachreagieren. Man wäscht das Rohprodukt zweimal mit gesättigter Natriumhydrogencarbonatlösung und einmal mit Wasser und erhält nach Destillation 153,2 g 3-Ethoxipropionsäure-tert.-butylester (Kp. 86°C/44 mbar).
$^1$H-NMR: δ = 1,45 ppm (s, 9H), 2,5 ppm (t,2H), 3,65 ppm (t,2H); zusätzlich: δ = 1,2 ppm (t,3H), 3,5 ppm (q,2H).

Beispiel 2

Benzyl-trimethylammoniumhydroxid-katalysierte Addition von Allylalkohol an Acrylsäure-tert.-butylester

Zum vorgelegten Gemisch von 58 g Allylalkoholol und 8 g Benzyl-trimethylammoniumhydroxid tropft man unter Rühren innerhalb von ca. 3 h bei 19-24°C 154 g Acrylsäure-tert.-butylester und läßt noch 5 h bei ca. 20°C nachreagieren. Nach der in Beispiel 1 beschriebenen Aufarbeitung erhält man 168,2 g 3-Allyloxipropionsäure-tert.-butylester (Kp. 55°C/13 mbar).
$^1$H-NMR: δ = 1,4 ppm (s,9H), 2,5 ppm (t,2H), 3,65 ppm (t,2H); zusätzlich: δ = 4,0 ppm (m,2H), 5,1-5,3 ppm (m,3H), 5,8-6,0 ppm (m,1H).

Beispiel 3

Durch in-situ hergestelltes Tetra-n-butylammoniumhydroxid katalysierte Addition von S-Ethylthioethanol an Acrylsäure-tert.-butylester

Zum vorgelegtem Gemisch aus 53 g S-Ethylthioethanol, 1,4 g festem Kaliumhydroxid und 1,6 g Tetra-n-butylammoniumbromid tropft man unter Rühren innerhalb von 2 h bei 20-22°C 70,4 g Acrylsäure-tert.-butylester und läßt noch 6 h bei ca. 20°C nachreagieren. Das Rohprodukt wird einmal mit dem gleichen Volumen Wasser gewaschen und i.Vak. getrocknet. Man erhält 109 g 3-(S-Ethylthioethoxi)-propionsäure-tert.-butylester.
$^1$H-NMR: δ = 1,45 ppm (s,9H), 2,5 ppm (t,2H); 3,6 ppm (t,2H); zusätzlich: δ = 1,25 ppm (t,3H); 2,6 ppm (g,2H), 2,7 ppm (t, 2H); 3,7 ppm (t,2H).

Beispiel 4

Benzyl-trimethylammoniumhydroxid-katalysierte, zweifache Addition von Butan-1,4-diol an Acrylsäure-tert.-butylester

Zum vorgelegten Gemisch aus 45 g Butan-1,4-diol und 4 g Benzyl-trimethylammoniumhydroxid tropft man unter Rühren innerhalb von 2 h bei 23°C 154 g Acrylsäure-tert.-butylester und läßt noch 8 h bei 20-22°C nachreagieren. Nach der in Beispiel 3 beschriebenen Aufarbeitung erhält man 156 g 3-(1-tert.-Butoxicarbonylethoxibutoxi)-propionsäure-tert.-butylester (Kp.: ~150°C/0,1 mbar).
$^1$H-NMR: δ = 1,45 ppm (s,18H), 2,45 ppm (t,4H), 3,65 ppm (t,4H); zusätzlich: δ = 1,6 ppm (m,4H), 3,4 ppm (m,4H).

Beispiel 5

Durch in-situ hergestelles Tetra-n-butylammoniumhydroxid katalysierte, zweifache Addition von Hexin-3-diol-2,5 an Acrylsäure-tert.-butylester.

Zum vorgelegten Gemisch aus 114 g Hexin-3-diol-2,5, 2,8 g festem Kaliumhydroxid und 3,2 g Tetra-n-butylammoniumbromid tropft man unter Rühren innerhalb von 2,5 h bei 22°C 281,6 g Acrylsäure-tert.-butylester und läßt nach 48 h bei 20-22°C nachrühren. Nach Wäsche mit jeweils dem gleichen Volumen gesättigter Natriumhydrogencarbonatlösung und Wasser und dem Trocknen i.Vak. erhält man 320,8 g 2,5-Bis-(tert.-butoxicarbonylethoxi)-but-3-in.
$^1$H-NMR: $\delta$ = 1,45 (s,18H), 2,5 ppm (t,4H), 3,6 und 3,9 ppm (m, je 2H; Aufspaltung beruht auf Vorhandensein eines Chiralitätszentrums!); zusätzlich: $\delta$ = 1,4 ppm (s,6H), 4,2 ppm (q, 2H).

Beispiel 6

Benzyl-trimethylammoniumhydroxid-katalysierte, dreifache Addition von Triethanolamin an Acrylsäure-tert.-butylester in THF-Lösung.

Zum vorgelegten Gemisch aus 50 g Tetrahydrofuran, 50 g Triethanolamin und 2,7 g Benzyl-trimethylammoniumhydroxid tropft man unter Rühren innerhalb von 2,5 h bei 21 °C 154 g Acrylsäure-tert.-butylester und läßt noch 10 h bei 20-22°C nachreagieren. Man zieht das Tetrahydrofuran bei max. 60°C am Rotationsverdampfer ab, wäscht den Rückstand zweimal mit dem gleichen Volumen gesättigter Natriumhydrogencarbonatlösung und trocknet den Rückstand i.Vak. Man erhält 170 g N,N,N-Tri-(tert.-butoxicarbonylethoxiethyl)-amin.
$^1$H-NMR: $\delta$ = 1,45 ppm (s,27H), 2,5 ppm (t, 6H), 3,65 ppm (t,6H); zusätzlich: $\delta$ = 2,75 ppm (t,6H), 3,5 ppm (t,6H).

Beispiele 7 bis 72

Die in den Tabellen 1 bis 7 aufgeführten Beispiele 7-72 wurden in Analogie zu Beispielen 1-6 durchgeführt. Die Reaktions-Rohprodukte wurden neutralisiert, mit Wasser gewaschen und unter reduziertem Druck von den Niedersiedern befreit.
In den Tabellen haben die "Katalysatorkomponenten" folgende Bedeutungen:

A: $(n\text{-}C_4H_9)_4N^+Br^-$

B: KOH-Pulver

C: $n\text{-}C_{12}H_{25}(CH_3)_3N^+Br^-$

D: 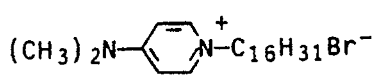

E: $n\text{-}C_{12}H_{25}(n\text{-}C_4H_9)_3P^+Br^-$

F: $(n\text{-}C_4H_9)_4P^+Br^-$

G: $C_6H_5\text{-}CH_2\text{-}N^+(CH_3)_3{}^-OH$ (Triton® B).

Tabelle 1

| Bei-spiel Nr. | Alkohol R | [g] | Acrylsäure-tert.-butylester [g] | Katalysator Kompo-nenten | [g] | Lösungs-mittel Typ | [g] | Temp. [°C] | Dauer [h] | Ausbeute [g] |
|---|---|---|---|---|---|---|---|---|---|---|
| 7 | $CH_3$- | 64 | 281,6 | A<br>B | 6,4<br>5,6 | - | - | 25 | 14 | 286,2 |
| 8 | $CH_3$- | 32 | 140,8 | C<br>B | 7,3<br>2,24 | - | - | 21-27 | 8 | 136,5 |
| 9 | $CH_3$- | 32 | 140,8 | D<br>B | 7,2<br>2,24 | - | - | 22-30 | 8 | 140,2 |
| 10 | $CH_3$- | 32 | 140,8 | E<br>B | 10,4<br>2,24 | - | - | 21-29 | 8 | 132,7 |
| 11 | $CH_3$- | 32 | 140,8 | F<br>B | 6,8<br>2,24 | - | - | 21-28 | 8 | 136,9 |
| 12 | n-$C_3H_7$- | 120 | 281,5 | A<br>B | 6,4<br>5,6 | - | - | 20-29 | 12 | 354,3 |
| 13 | i-$C_3H_7$- | 13,2 | 25,6 | A<br>B | 3,2<br>2,8 | THF | 10 | 25-33<br>70 | 14<br>3 | 31,5 |
| 14 | n-$C_4H_9$- | 74 | 154 | G | 8,0 | - | - | 23-25 | 40 | 179,4 |
| 15 | i-$C_4H_9$- | 74 | 140,8 | A<br>B | 3,2<br>2,8 | - | - | 22-25 | 18 | 169,8 |
| 16 | sec-$C_4H_9$- | 74,0 | 140,8 | G<br>A<br>B | 8,0<br>3,2<br>2,8 | - | - | 24-28 | 18 | 169,6 |
| 17 | n-$C_5H_{11}$- | 71,2 | 112,6 | G | 8,0 | - | - | 20-23 | 16 | 186,0 |

Tabelle 1 (Fortsetzung)

| Beispiel Nr. | Alkohol R | Alkohol [g] | Acrylsäure-tert.-butylester [g] | Katalysator Komponenten | Katalysator [g] | Lösungsmittel Typ | Lösungsmittel [g] | Temp. [°C] | Dauer [h] | Ausbeute [g] |
|---|---|---|---|---|---|---|---|---|---|---|
| 18 | H<br>(S)-$CH_3$-$CH_2$-C-$CH_2$-<br>$CH_3$ | 78 | 124,8 | A<br>B | 6,0<br>5,3 | - | - | 25-30 | 24 | 174,4 |
| 19 | $(CH_3)_3C-CH_2-$ | 88,0 | 140,8 | G | 8,0 | THF | 88 | 26-28 | 14 | 179,1 |
| 20 | $CH_3-(CH_2)_3-CH-CH_2-$<br>$CH_2-CH_3$ | 130,0 | 141 | G | 10,0 | - | - | 20-23 | 12 | 230 |
| 21 | Cyclopropylmethyl | 14,4 | 28,2 | A<br>B | 0,64<br>0,56 | THF | 14 | 21-22 | 12 | 29,8 |
| 22 | Cyclobutyl | 43,5 | 70,4 | A<br>B | 1,6<br>1,4 | - | - | 21-30 | 12 | 79,7 |
| 23 | Cyclohexyl | 80 | 122 | G<br>A<br>B | 6,4<br>6,4<br>5,6 | THF | 80 | 70 | 13 | 135,8 |
| 24 | 3-Methyl-but-2--en-1-yl | 43,0 | 70,4 | A<br>B | 1,6<br>1,4 | - | - | 24 | 24 | 93,4 |
| 25 | Hex-5-en-1-yl | 50 | 76,8 | G | 4,0 | - | - | 21-47 | 12 | 104,2 |
| 26 | Oct-7-en-1-yl | 64 | 76,8 | A<br>B | 1,6<br>1,4 | - | - | 26-33 | 8 | 110,6 |
| 27 | Dec-9-en-1-yl | 78 | 70,4 | A<br>B | 1,6<br>1,4 | - | - | 21-28 | 10 | 125,1 |

Tabelle 1 (Fortsetzung)

| Beispiel Nr. | Alkohol R | Alkohol [g] | Acrylsäure-tert.-butylester [g] | Katalysator Komponenten | Katalysator [g] | Lösungsmittel Typ | Lösungsmittel [g] | Temp. [°C] | Dauer [h] | Ausbeute [g] |
|---|---|---|---|---|---|---|---|---|---|---|
| 28 | 3,7-Dimethyl-oct-6-en-1-yl | 78,2 | 70,5 | G | 5,0 | - | - | 22-26 | 8 | 115,5 |
| 29 | 3,7-Dimethyl-oct-2,6-dien-1-yl | 154,2 | 154 | G | 8,0 | - | - | 24-28 | 4 | 259 |
| 30 | Benzyl | 108 | 154 | G | 8,0 | - | - | 20-21 | - | 180,2 |
| 31 | Phenethyl | 122 | 141 | A<br>B | 3,2<br>1,4 | - | - | 26 | 18 | 194,9 |
| 32 | 4-Chlorbenzyl | 71,3 | 70,4 | A<br>B | 1,6<br>1,4 | $CH_3CN$ | 120 | 21-28 | 16 | 126,6 |
| 33 | 4-Methoxybenzyl | 69 | 70,4 | A<br>B | 1,6<br>1,4 | - | - | 26-27 | 22 | 105,3 |
| 34 | 4-Nitrobenzyl | 153,1 | 140,8 | A<br>B | 3,2<br>2,8 | THF | 180 | 20-30 | 8 | 226,0 |
| 35 | 4-Methoxyphenethyl | 152 | 140,8 | G | 8,0 | - | - | 24-46 | 12 | 220,7 |
| 36 | 4-Phenyl-prop-2-en-1-yl | 136,7 | 141 | A<br>B | 3,2<br>2,8 | THF | 178 | 26 | 4 | 231,2 |
| 37 | $CF_3-CH_2-$ | 50,5 | 70,4 | A<br>B | 1,6<br>1,4 | - | - | 20-27 | 10 | 98,1 |
| 38 | $Cl-(CH_2)_6-$ | 67,3 | 70,4 | A<br>B | 1,6<br>1,4 | - | - | 25-38 | 24 | 88,3 |
| 39 | $CH_3O-CH_2-CH_2-$ | 76 | 140,8 | G | 10 | - | - | 25-38 | 8 | 194 |

Tabelle 1 (Fortsetzung)

| Beispiel Nr. | Alkohol R | [g] | Acrylsäure-tert.-butylester [g] | Katalysator Komponenten | Katalysator [g] | Lösungsmittel Typ | Lösungsmittel [g] | Temp. [°C] | Dauer [h] | Ausbeute [g] |
|---|---|---|---|---|---|---|---|---|---|---|
| 40 | $CH_3-CH(OCH_3)-CH_2-$ | 90 | 140,8 | A<br>B | 3,2<br>2,8 | - | - | 21-24<br>60 | 8<br>4 | 120 |
| 41 | $H_2C=CH-O-(CH_2)_2-O-(CH_2)_2-$ | 792 | 922 | G | 24 | - | - | 22-27 | 6 | 146,6 |
| 42 | Phenethoxyethyl | 122 | 141 | A<br>B | 3,2<br>2,8 | - | - | 26 | 8 | 194,9 |
| 43 | $CH_2(SH)-CH_2-$ | 39 | 153,6 | G | 8,0 | - | - | 23-36 | 24 | 140,6 |
| 44 | $(CH_3)_2N-CH_2-CH_2-$ | 89 | 140,8 | A<br>B | 3,2<br>2,8 | - | - | 25-26 | 8 | 182,3 |
| 45 | $(CH_3)_2N-(CH_2)_3-$ | 103 | 140,8 | A<br>B | 3,2<br>2,8 | - | - | 25-30 | 10 | 198,6 |
| 46 | $(CH_3)_2N-(CH_2)_2-O-(CH_2)_2-$ | 89 | 140,8 | A<br>B | 3,2<br>2,8 | - | - | 25-26 | 18 | |
| 47 | $CH_3N(CH_2-CH_2-)(CH_2-CH_2-COOC(CH_3)_3)$ | 200 | 154 | G | 8,0 | - | - | 21-23 | 12 | 275 |
| 48 | $C_6H_5-N(CH_2-CH_3)-CH_2-CH_2-$ | 83,5 | 70,4 | G | 5,0 | - | - | 20-23 | 12 | 130 |

Tabelle 1 (Fortsetzung)

| Beispiel Nr. | Alkohol R | Alkohol [g] | Acrylsäure-tert.-butylester [g] | Katalysator Komponenten | Katalysator [g] | Lösungsmittel Typ | Lösungsmittel [g] | Temp. [°C] | Dauer [h] | Ausbeute [g] |
|---|---|---|---|---|---|---|---|---|---|---|
| 49 | $NC-(CH_2)_6-$ | 63,5 | 70,4 | A<br>B | 1,6<br>1,4 | - | - | 24-26 | 12 | 108,1 |
| 50 | $(CH_3)_3Si-CH_2-CH_2-$ | 11,8 | 14,1 | G | 0,8 | - | - | 20-22 | 24 | 23,2 |
| 51 | Tetrahydrofuran-2-methyl | 102 | 154 | G | 8,0 | - | - | 25-32 | 18 | 211 |
| 52 | 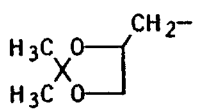 | 52,8 | 56,3 | A<br>B | 1,28<br>1,12 | - | - | 22-25<br>40-50 | 12<br>3 | 79,6 |
| 53 | Furan-2-methyl | 50,0 | 70,5 | G | 9,0 | - | - | 22-30 | 12 | 98,7 |
| 54 | 2-(5-Formyl-tetrahydrofuran-2)-ethyl | 25,2 | 31,0 | G | 1,6 | $CH_3CN$ | 27,4 | 21-23 | 24 | 49,0 |
| 55 | Furan-2-methyl | 114 | 140,8 | G | 8,5 | - | - | 21-24 | 10 | 215,4 |
| 56 | 2-(N-Morpholino)ethyl | 104,8 | 112,6 | G | 8,0 | - | - | 20-23 | 12 | 179,7 |
| 57 | 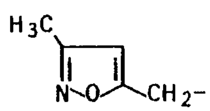 | 11,8 | 15,4 | G<br>A<br>B | 0,8<br>0,32<br>0,28 | - | - | 22-23 | 24 | 16,2 |
| 58 | Pyridin-2-methyl | 110 | 140,8 | G | 8,5 | - | - | 21-23 | 12 | 204,0 |
| 59 | $CH_3-CH_2-CH(OH)-CH_2-$ | 90,0 | 140,8 | A<br>B | 3,2<br>2,8 | - | - | 23-24 | 60 | 102,6 |

Tabelle 1 (Fortsetzung)

| Beispiel Nr. | Alkohol R | Alkohol [g] | Acrylsäure-tert.-butylester [g] | Katalysator Komponenten | Katalysator [g] | Lösungsmittel Typ | Lösungsmittel [g] | Temp. [°C] | Dauer [h] | Ausbeute [g] |
|---|---|---|---|---|---|---|---|---|---|---|
| 60 | 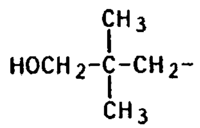 | 52,0 | 140,8 | A<br>B | 1,6<br>1,4 | THF | 52 | 20-22 | 36 | 63,7 |
| 61 | 4-Hydroxy-but-2-en--1-yl | 44,0 | 140,8 | A<br>B | 1,6<br>1,4 | - | - | 22-24 | 24 | 42,4 (Mono-Add.)<br>77,6 (Di-Addukt) |
| 62 | 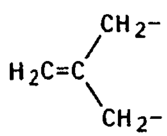 | 78 | 251 | A<br>B | 3,2<br>2,8 | THF | 45 | 23-25 | 36 | 115,0 (Mono-Add.)<br>11,5 (Di-Addukt) |
| 63 | 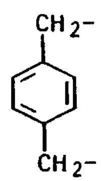 | 59 | 132 | G | 4,0 | THF | 180 | 22-40 | 12 | 157 (Mono-Add.)<br>11,5 (Di-Addukt) |
| 64 | 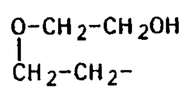 | 106 | 281,6 | G<br>A<br>B | 2,0<br>3,2<br>2,8 | - | - | 27-32 | 36 | 93,9 (Mono-Add.) |

Tabelle 1 (Fortsetzung)

| Beispiel Nr. | Alkohol R | Alkohol [g] | Acrylsäure-tert.-butylester [g] | Katalysator Komponenten | Katalysator [g] | Lösungsmittel Typ | Lösungsmittel [g] | Temp. [°C] | Dauer [h] | Ausbeute [g] |
|---|---|---|---|---|---|---|---|---|---|---|
| 65 | $-CH_2-CH_2-[O-CH_2-CH_2-]_n$<br>$\bar{n} \approx 8,7$<br>"Pluriol E 400" | 400 | 308 | G | 16 | - | - | 25-32 | 36 | 634,3 |
| 66 | $-CH_2-CH_2-[O-CH_2-CH_2-]_n$<br>$\bar{n} \approx 33,7$<br>"Pluriol E 1500" | 150 | 32 | G | 1,6 | THF | 150 | 23-35 | 36 | 165,1 |
| 67 | $-CH(CH_3)-CH_2-[O-CH(CH_3)-CH_2-]_n$<br>$\bar{n} \approx 34,2$<br>"Pluriol P 2000" | 20 | 3,1 | A<br>B | 0,032<br>0,028 | THF | 20 | 22-24 | 36 | 21,5 |
| 68 | $-CH(R)-CH_2-[O-CH(R)-CH_2-]_n$<br>R=H (ca. 40 mol-%)<br>=$CH_3$ (ca. 60 mol-%)<br>$\bar{n} \approx 120$<br>"Pluriol PE 6100" | 122 | 12,3 | G<br>A<br>B | 1,6<br>0,064<br>0,056 | - | - | 23 | 36 | 129,0 |
| 69 | $S-(-CH_2-CH_2-)_2$ | 123 | 282 | A<br>B | 3,2<br>2,8 | - | - | 22-26 | 10 | 319,1 |
| 70 | $CH_3N-(CH_2-CH_2-)_2$ | 119 | 281,6 | A<br>B | 3,2<br>2,8 | - | - | 24-26 | 6 | 291,9 |

Tabelle 1 (Fortsetzung)

| Bei-spiel Nr. | Alkohol R | [g] | Acrylsäure-tert.-butylester [g] | Katalysator Kompo-nenten | Katalysator [g] | Lösungs-mittel Typ | Lösungs-mittel [g] | Temp. [°C] | Dauer [h] | Ausbeute [g] |
|---|---|---|---|---|---|---|---|---|---|---|
| 71 | $C_6H_5-N-(-CH_2-CH_2-)_2$ | 184,6 | 281,6 | G | 8,0 | THF | 185 | 25-35 | 8 | 369,2 |
| 72 | 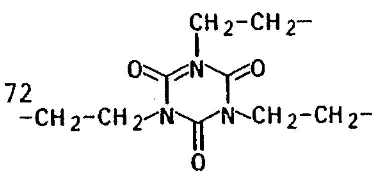 | 26,5 | 46 | G | 1,6 | NMP | 175 | 22-40 | 48 | 31 |

## Ansprüche

1. Substituierte 3-Oxipropionsäure-tert.-butylester der allgemeinen Formel I

$R[-O-(CH_2)_2-COOC(CH_3)_3]_n$ (I)

in der

R Alkyl mit 2, 3 oder 5 bis 20 C-Atomen, Alkandiyl mit 2 bis 20 C-Atomen, Alkantriyl mit 3 bis 20 C-Atomen, Alkantetryl mit 4 bis 20 C-Atomen, Alkenyl mit 3 bis 20 C-Atomen, Alkendiyl mit 4 bis 20 C-Atomen, Alkinyl mit 3 bis 20 C-Atomen, Alkindiyl mit 4 bis 20 C-Atomen, Polyether-α,ω-diyl der allgemeinen Formel

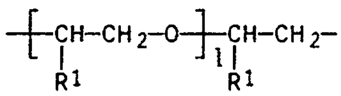

(II)

in der $R^1$ für Wasserstoff und/oder Methyl und l für eine ganze Zahl von 4 bis 400 steht, bedeutet, wobei die Reste R durch ein bis fünf Substituenten aus der Gruppe Halogen, Hydroxi, Alkoxi, Alkoxialkyl, Acyloxi, Trialkylsilyl, Thiol, Thio, sec.-Amino, tert.-Amino, Oximether, Nitro, Halogenalkyl, Aldehyd, Acyl, Ketal, Ester, Nitril, gegebenenfalls substituiertes Aryl und aromatische oder nichtaromatische Heterocyclen substituiert sein können, und

n 1 bis 4 bedeutet.

2. Verfahren zur Herstellung von substituierten 3-Oxipropionsäure-tert.-butylestern nach Anspruch 1, dadurch gekennzeichnet, daß man Alkohole der Formel R-OH, in der R die in Anspruch 1 genannte Bedeutung hat, in Gegenwart von basischen Katalysatoren mit Acrylsäure-tert.-butylester umsetzt.

3. Verfahren zur Herstellung von substituierten 3-Oxipropionsäure-tert.-butylestern nach Ansprüchen 1 und 2, dadurch gekennzeichnet, daß man als basische Katalysatoren Gemische aus anorganischen Basen und quartären Ammonium- oder quartären Phosphonium-verbindungen einsetzt.

4. Verfahren zur Herstellung von substituierten 3-Oxipropionsäure-tert.-butylestern nach den Ansprüchen 1 bis 3, dadurch gekennzeichnet, daß man quartäre Ammonium- oder quartäre Phosphonium-hydroxide einsetzt.

Europäisches Patentamt

# EUROPÄISCHER RECHERCHENBERICHT

Nummer der Anmeldung

**EP 90 11 3915**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 254 291 (UNION CARBIDE)<br>* Seite 13, Patentansprüche *<br>----- | 1 | C 07 C 69/708<br>C 07 C 67/31 |

| RECHERCHIERTE SACHGEBIETE (Int. Cl.5) |
|---|
| C 07 C 69/00<br>C 07 C 67/00 |

Der vorliegende Recherchenbericht wurde für alle Patentansprüche erstellt

| Recherchenort | Abschlußdatum der Recherche | Prüfer |
|---|---|---|
| Den Haag | 24 Oktober 90 | KINZINGER J.M. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument